# EUROPÄISCHE PATENTSCHRIFT

(11) **EP 2 497 428 B1**
(45) Veröffentlichungstag und Bekanntmachung des Hinweises auf die Patenterteilung: **11.02.2015**
(21) Anmeldenummer: 11157926.4
(22) Anmeldetag: 11.03.2011
(51) Int. Cl.: A61B 17/00, A61B 17/29, A61B 17/30, A61B 17/28, F16C 11/12

(54) **Nachgiebige Werkzeuge**
Flexible tools
Outils flexibles

(43) Veröffentlichungstag der Anmeldung: 12.09.2012
(73) Patentinhaber: EMPA Eidgenössische Materialprüfungs- und Forschungsanstalt, 8600 Dübendorf (CH)
(72) Erfinder: Campanile, Lucio Flavio, 8603, Schwerzenbach (CH); Hasse, Alexander, 01445, Radebeul (DE)
(74) Vertreter: Schulz Junghans Patentanwälte PartGmbB

(56) Entgegenhaltungen:
- BE-A1- 814 069
- FR-A1- 2 914 713
- NL-C2- 1 018 160
- US-A- 4 571 988

## Beschreibung

### Technisches Gebiet

Die Erfindung betrifft nachgiebige Werkzeuge, insbesondere Hebel zum Übertragen von Kräften und/oder Bewegungen gemäss dem Oberbegriff von Anspruch 1.

### Stand der Technik

Anordnungen, bei denen nachgiebige Bauelemente verwendet werden, um eine Kraftübertragung zwischen z.B. zwei Hebelarmen bzw. eine Bewegung solcher Arme zu ermöglichen sind seit langem bekannt.

Ein typischer Einsatz für solche Anordnungen ist ein mikromechanisches Biegefedergelenk, wie es bei Beschleunigungsmessern oder dynamisch abgestimmten mechanischen Kreiseln zum Einsatz kommt und aus der EP 0 275 338 A1, der US 4 261 211 A, der EP 1 887 398 A1 oder der DE 2 653 427 A1 bekannt ist. Eine ähnliche Anordnung ist aus der FR 2 666 630 A1 oder der US 5 620 169 A1 bekannt. Diesen Baugruppen ist ein hoher Grad an Symmetrie zueigen, der sich aus dem Einsatz ergibt. Diese Anwendungen sind allerdings nicht planar, sondern führen zu Bewegungen im dreidimensionalen Raum, was im vorliegenden Fall nicht die Aufgabe der Erfindung ist.

Grundsätzlich bekannt ist auch, dass nachgiebige Verbindungen für Werkzeuge eingesetzt werden können, wie z.B. aus der US 3 906 957 A oder der US 4 526 172 A. Auch diesen Werkzeugen ist ein gewisser Grad an Symmetrie zueigen, der aus der herkömmlichen Vorlage der entsprechenden Werkzeuge herrührt und nicht überwunden werden sollte. Solche Werkzeuge können als planare Anwendungen angesehen werden, bei denen die Bewegung grundsätzlich in einer Ebene stattfindet. Derartige planare Werkzeuganwendungen weisen - gegenüber herkömmlichen Mechanisierungen mit einem Gelenk etc. - bereits zahlreiche Vorteile auf, wie z.B. bessere Abnutzungseigenschaften, die Vermeidung von Rückpralleffekten, die Vermeidung bzw. die Verringerung von Einsatzgeräuschen, die Vermeidung des Freisetzens von Teilchen etc., die einfache Wartung, Reinigung und Herstellung, die höhere Präzision. Die einfachere Wartung und Reinigung kann insbesondere bei chirurgischen Instrumenten eine essentielle Eigenschaft sein, wenn sie z.B. die Desinfektion einschliesst. Dazu kommt in diesem Fall noch das Fehlen von Zwischenräumen, die zu Verletzungen führen können sowie die Möglichkeit, einfachere Entsorgung zu ermöglichen.

Weitere Anordnungen sind aus der NL 1018 160 C2 und der FR 2 914 713 A1 bekannt.

Allerdings weisen die nachgiebigen Werkzeuge gemäss dem Stand der Technik einige Nachteile auf. Insbesondere scheinen die Anforderungen an ein einfaches Layout, gute Verformbarkeit bei gleichzeitig einer kräftigen Auslegung des Werkzeugs, geringe Anforderungen an das Material und ein geringes Gewicht mit den im Stand der Technik beschriebenen Werkzeuge noch nicht optimal zu sein.

### Darstellung der Erfindung

Die Erfindung wird in Anspruch 1 beansprucht.

Aufgabe der Erfindung ist es, nachgiebige Werkzeuge, bei denen prinzipiell zwei - oder mehr - feste, steife Bauelemente, wie beispielsweise die Hebel und Griffe einer Zange, als Ersatz für ein Gelenk mittels nachgiebiger Elemente miteinander verbunden sind, in dem oben genannten Sinne zu verbessern.

Die Aufgabe der Erfindung wird durch einen Handgriff nach Anspruch 1 gelöst. Dabei haben die Massnahmen der Erfindung zunächst einmal zur Folge, dass dadurch, dass sich die Mittellinien der Verbindungselemente in einem Punkt schneiden und der Schnittpunkt auf der Oberfläche eines ersten der steifen Bauelemente oder in einem kleinen Abstand dazu liegt, eine optimale Ausnutzung der geometrischen Verhältnisse ermöglicht wird.

Nachgiebige Mechanismen realisieren die geforderte Bewegung über die elastische Deformation von Strukturkomponenten. Bei ihrem Design müssen hauptsächlich folgende Aspekte beachtet werden:
1. Der Mechanismus muss in der Lage sein, die von ihm geforderte Bewegung ohne Schaden zu überstehen.
2. Der Mechanismus muss in der Lage sein, Betriebslasten ohne Schaden zu ertragen.
3. Der Mechanismus muss eine bestimmte Steifigkeit in der bevorzugten Bewegungsrichtung aufweisen (vergleichbar mit einer Rückstellkraft).
4. Der Mechanismus sollte in alle Richtungen, welche von der bevorzugten Bewegungsrichtung abweisen, eine möglichst hohe Steifigkeit haben, um so ungewollte Verformungen zu vermeiden.

Die in der vorliegenden Erfindung vorgestellte Auslegung begünstigt vor allem die Aspekte 2 und 4. Die nachgiebigen Verbindungselemente interagieren durch die vorgestellte Anordnung, so dass sie einen Bewegungsfreiheitsgrad des Mechanismus ermöglichen, aber Verformungen, die nicht diesen Bewegungsfreiheitsgrad betreffen, durch eine sehr hohe Struktursteifigkeit behindern (Aspekt 4). Das beschriebene Strukturverhalten hat impliziert positive Auswirkungen auf Aspekt 2, da die Deformation der nachgiebigen Verbindungselemente fast unabhängig von den auftretenden Operationslasten gegeben ist.

Die Designanforderungen (Aspekte 2 und 4) werden ideal begünstig, wenn sich infinitesimal dünne Verbindungselemente genau in ihrem Schnittpunkt mit der Oberfläche des im Wesentlichen steifen Bauelements verbunden sind. Dies ist allerdings bei Betrachtung von Realstrukturen nicht möglich. Die Verbindungselemente sind immer mit finiten Dicken versehen, so dass sich der Schnittpunkt der Mittellinien der Verbindungselemente immer in einem kleinen Abstand zur Oberfläche der im Wesentlichen steifen Bauelemente befindet.

Die positive Auswirkung des Designs auf die Aspekte 2 und 4 bleibt für gewöhnlich erhalten, wenn der Abstand des Schnittpunkts die zehnfache dünnste Breite der Verbindungselemente nicht überschreitet. Je geringer der Abstand umso besser die Auswirkung auf Aspekt 2 und 4.

Bei der Bestimmung der Form der nachgiebigen Verbindungselemente sind alle Aspekte 1 bis 4 zu berücksichtigen.

Eine weitere Abgrenzung zu bereits bestehenden Gelenkkonfigurationen (wie beispielsweise dem aus der DE 2 653 427 A1 bekannten Gelenk) ist es, dass eine Kinematik realisiert werden kann, bei der sich der Drehpunkt der im Wesentlichen steifen Bauelemente zueinander an der Oberfläche eines der im Wesentlichen steifen Bauelemente oder zumindest in einem kleinen Abstand dazu befindet.

Vorteilhaft ist es, wenn erfindungsgemäß der genannte Schnittpunkt der Mittellinien der Verbindungselemente - wenn schon nicht genau auf der Oberfläche so doch in einem Abstand von maximal dem zehnfachen der dünnsten Breite der Verbindungselemente, vorzugsweise dem fünffachen der dünnsten Breite der Verbindungselemente, höchst vorzugsweise dem doppelten der dünnsten Breite der Verbindungselemente zum ersten Bauelement liegt.

Eine andere vorteilhafte Ausgestaltung liegt vor, wenn der Schnittpunkt der Verbindungselemente zwischen den zwei steifen Bauelementen liegt und die Verbindung zwischen dem Schnittpunkt der Verbindungselemente und einem Bauelement im Wesentlichen steif ausgestaltet ist.

Vorteilhaft ist es, wenn der genannte Schnittpunkt der Mittellinien der Verbindungselemente in einem Abstand von maximal dem Zehnfachen der dünnsten Breite der Verbindungselemente, vorzugsweise dem Fünffachen der dünnsten Breite der Verbindungselemente, höchst vorzugsweise dem doppelten der dünnsten Breite der Verbindungselemente zum ersten Bauelement oder aber im Bereich des ersten Bauelementes liegt.

In einer Weiterentwicklung des erfindungsgemässen Werkzeugs sind weitere nachgiebige Verbindungselemente vorgesehen. Dabei bilden jeweils zwei der Verbindungselemente mit einem Teilstück eines der Bauelemente derart ein Dreieck, dass eine der Ecken des Dreieckes im Bereich des anderen Bauelementes oder zumindest in einem kleinen Abstand liegt, wiederum normalerweise in einem Abstand von maximal dem zehnfachen der dünnsten Breite der Verbindungselemente, vorzugsweise dem fünffachen der dünnsten Breite der Verbindungselemente, höchst vorzugsweise dem doppelten der dünnsten Breite der Verbindungselemente zum ersten Bauelement.

In diesem Sinne sind die Eckpunkte der Dreiecke Synonyme für die Schnittpunkte gemäss Anspruch 1. Durch diese Beschreibung wird also kein geänderter Sachverhalt beschrieben. Vielmehr soll angedeutet werden, dass die Dreiecke auch solche mit nicht geraden Kanten einschliessen sollen, wie sie z.B. als Kugeldreiecke der sphärischen Trigonometrie bekannt sind.

Als ganz besonders vorteilhaft hat sich der Einsatz eines solchen Werkzeuges herausgestellt, wenn der Hebel erfindungsgemäß als Handgriff für die minimal invasive, insbesondere für die laparoskopische Chirurgie ausgebildet ist. Die Bewegung des Hebels gemäss diesem Aspekt der Erfindung wird mittels einer Zug-/Druckstange - ganz allgemein durch ein geeignetes Übertragungselement - an das so genannte "distale" Ende des Instruments übertragen, das sich während des chirurgischen Eingriffs im menschlichen Körper befindet. Das "distale" Ende kann je nach Zweck verschiedene Formen annehmen. Typische distale Enden sind Nadelhalter, Scheren oder Zangen zur Entnahme von Gewebeproben.

Als stoffschlüssige Verbindungen werden im Sinne dieser Schrift alle Verbindungen angesehen, bei denen die Verbindungspartner durch atomare oder molekulare Kräfte zusammengehalten werden. Darunter fallen - neben der Herstellung aus einem Stück - alle nicht lösbaren Verbindungen, die sich nur durch Zerstörung der Verbindungsmittel trennen lassen, einschliesslich Verbindungen durch Löten, Schweissen, Kleben, allenfalls auch Vulkanisieren etc.

Die vorbenannten sowie die beanspruchten und in den nachfolgenden Ausführungsbeispielen beschriebenen, erfindungsgemäss zu verwendenden Elemente unterliegen in ihrer Grösse, Formgestaltung, Materialverwendung und ihrer technischen Konzeption - über die Merkmale der Patentansprüche - keinen besonderen Ausnahmebedingungen, so dass die in dem jeweiligen Anwendungsgebiet bekannten Auswahlkriterien uneingeschränkt Anwendung finden können.

### Kurze Beschreibung der Zeichnungen

Weitere Einzelheiten, Vorteile und Merkmale des Gegenstandes der vorliegenden Erfindung ergeben sich aus der nachfolgenden Beschreibung der dazu gehörenden Zeichnungen, in denen - beispielhaft - erfindungsgemässe Vorrichtungen erläutert werden. In den Zeichnungen zeigt:
- Figur 1: eine Grundform eines Werkzeugs mit zwei steifen Bauelementen, die mittels zweier nachgiebiger Verbindungselemente stoffschlüssig miteinander sind;
- Figur 2: eine erste Variation des Werkzeugs mit zwei steifen Bauelementen, die mittels dreier nachgiebiger Verbindungselemente stoffschlüssig miteinander sind;
- Figur 3: eine zweite Variation des Werkzeugs mit zwei steifen Bauelementen, die ebenfalls mittels dreier nachgiebiger Verbindungselemente stoffschlüssig miteinander sind;
- Figur 4: eine weitere Variante des Werkzeugs mit zwei steifen Bauelementen, die mittels zweier nachgiebiger Verbindungselemente stoffschlüssig miteinander sind, wobei der Schnittpunkt der beiden Verbindungselemente auf einem vorgeschobenen Bereich des einen Bauelementes liegt; und
- Figur 5: eine Variante des Werkzeugs gemäss Figur 4.

### Wege zur Ausführungder Erfindung

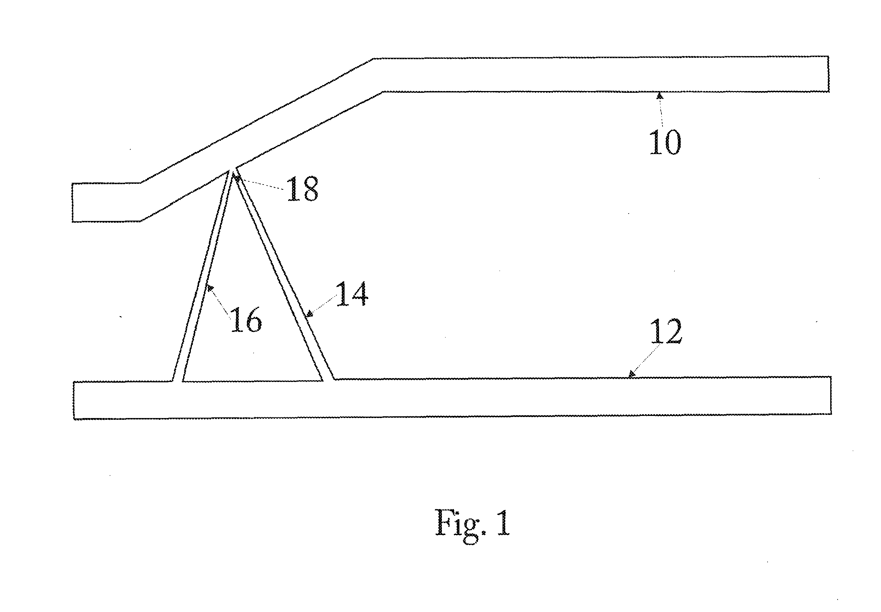

In Figur 1 ist ein einfaches Ausführungsbeispiel eines Werkzeuges gemäss der vorliegenden Erfindung dargestellt. Das Werkzeug ist als Hebelwerkzeug mit zwei als Hebel ausgebildeten, im Wesentlichen steifen Bauelementen 10 und 12 dargestellt, wobei die genaue Wirkweise der Hebel nicht gesondert dargestellt wird, da sie herkömmlich oder auch - dem Einsatzbereich des Werkzeuges gemäss - speziell sein kann. Im vorliegenden Ausführungsbeispiel könnte das Werkzeug so ausgebildet sein, dass die längeren Teilstücke der steifen Bauelemente zum - beispielsweise händischen - Zusammendrücken vorgesehen sind und die kürzeren Teilstücke zum - durch die Hebelwirkung verstärkten - Auseinaderdrücken, wobei auf die Darstellung von Hilfsmitteln, wie Handgriffe etc. verzichtet wurde.

Das dargestellte Werkzeug kann - mittels entsprechender Hilfsmittel - als Greifwerkzeug oder als Haltewerkzeug ausgebildet sein. In diesem Fall könnten die beiden Bauelemente 10 und 12 sich - durch eine Auslegung der Verbindungselemente 14 und 16 - an einer Seite berühren, solange keine Kraft ausgeübt wird.

Die beiden Bauelemente sind stoffschlüssig mittels zweier nachgiebiger Verbindungselemente 14 und 16 miteinander verbunden. Im vorliegenden Ausführungsbeispiel bilden die Verbindungselemente 14 und 16 mit einem Teilbereich des steifen Bauelementes 12 ein Dreieck, dessen eine Ecke, nämlich der Schnittpunkt der beiden Verbindungselemente, auf der Oberfläche des Bauelementes 10 zusammenfällt. Dieses Zusammenfallen ist nicht notwendigerweise exakt, sondern kann auch in einem kleinen Abstand zur Oberfläche ausgebildet sein. Dabei wird dieser Abstand normalerweise nicht grösser sein als das Zehnfache der geringsten Breite der nachgiebigen Verbindungselemente 12 und 14, vorzugsweise nicht mehr als das Fünffache und höchst vorzugsweise nicht mehr als das Doppelte. Dabei kann dieser kleine Abstand sowohl nach aussen, also vom Bauelement 10 weg als auch in das Bauelement 10 hinein ausgebildet sein. In dem Fall, dass der kleine Abstand nach aussen ausgebildet ist, kann - als besondere Ausgestaltung - vorgesehen sein, die Teilbereiche vom Schnittpunkt, also der Ecke 18 des Dreieckes bis zur Oberfläche des Bauelementes 10, nicht nachgiebig, sondern steif auszubilden, da die Nachgiebigkeit in diesem Bereich nicht erwünscht sein könnte.

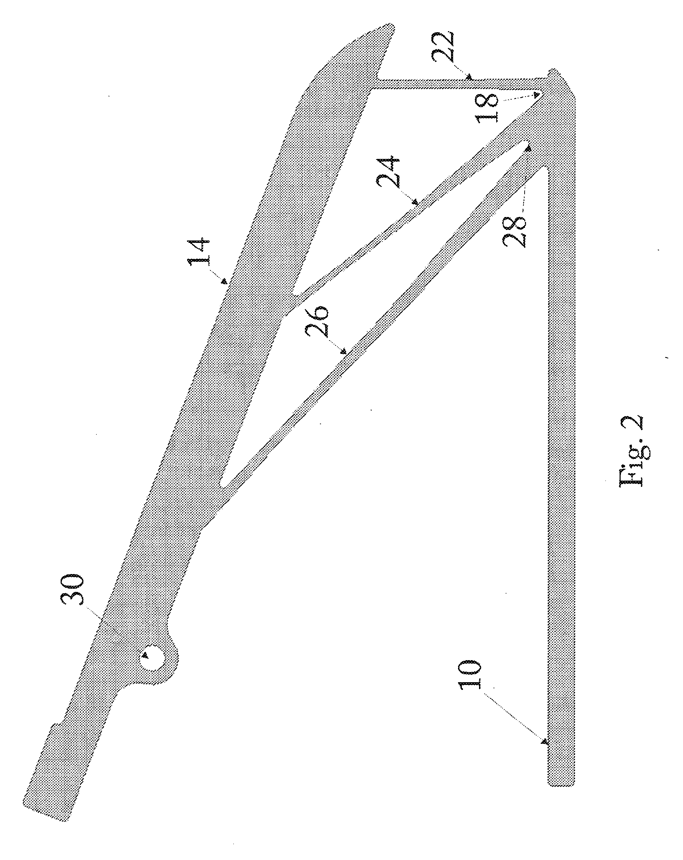

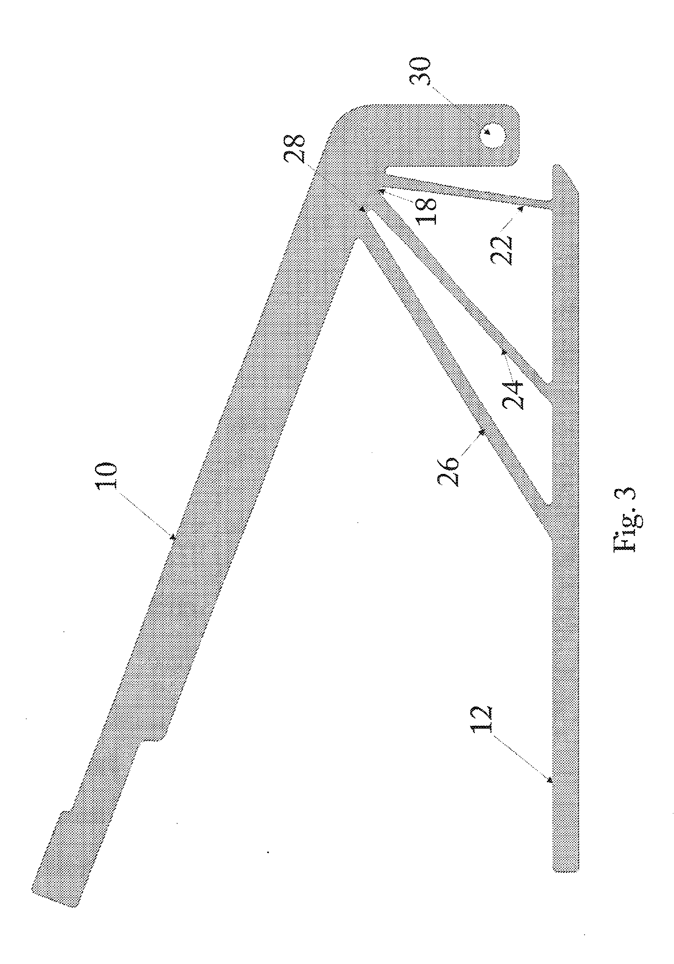

In einer alternativen Ausgestaltung der Erfindung sind nicht nur zwei nachgiebige Verbindungselemente vorgesehen, sondern eine Vielzahl davon. In dem in den Figuren 2 und 3 dargestellten Ausführungsbeispiel sind drei solche Verbindungselemente 22, 24 und 26 dargestellt. Dabei sind jeweils zwei der Verbindungselemente 22, 24, 26 mit einem Teilstück des Bauelementes 10 derart verbunden, dass sie - wiederum jeweils mit einem Teilstück des Bauelementes 12 - ein Dreieck bilden. Jeweils eine der beiden Ecken 18, 28 der Dreiecke liegen auf der Oberfläche des Bauelementes 10 oder in einem kleinen Abstand dazu. Auch hier ist dieses Zusammenfallen nicht notwendigerweise exakt, sondern kann auch in einem kleinen Abstand zur Oberfläche ausgebildet sein. Wiederum wird der Abstand normalerweise nicht grösser sein als das Zehnfache der geringsten Breite der nachgiebigen Verbindungselemente 22, 24, bzw. 26, vorzugsweise nicht mehr als das Fünffache und höchst vorzugsweise nicht mehr als das Doppelte. Dabei kann dieser kleine Abstand sowohl nach aussen, also vom Bauelement 10 weg als auch in das Bauelement 10 hinein ausgebildet sein.

Die Ausführungsform gemäss den Figuren 2 und 3 und auch Ausführungsformen mit mehr als 3 nachgiebigen Verbindungselementen zeichnen sich dadurch aus, dass die Kraft- und Hebelwirkungen sowie die Bewegungskurven zusammen mit der Materialauswahl der Verbindungselemente, die gleiche wie auch unterschiedliche Materialien wie die Bauelemente 10 und 12 aufweisen können, optimiert an die Anforderungen an das spezifische Werkzeug angepasst werden können. Als Materialien kommen - ohne Beschränkung darauf - Metalle mit elastischen Eigenschaften sowie Kunststoffe mit solchen Eigenschaften, aber auch keramische Werkstoffe oder Kombinationen davon oder ähnliches zum Einsatz.

In den Figuren 2 und 3 sind Bohrungen 30 - jeweils an unterschiedlichen Stellen - dargestellt, in denen weitere Mittel aufgenommen werden können, um das Werkzeug den Anforderungen und Gegebenheiten anzupassen, bei chirurgischen Instrumenten z.B. einen Faden, aber auch beliebige andere Mittel, die für Werkzeuge bekannt sind, ohne den Einsatzbereich der vorliegenden Erfindung zu verlassen.

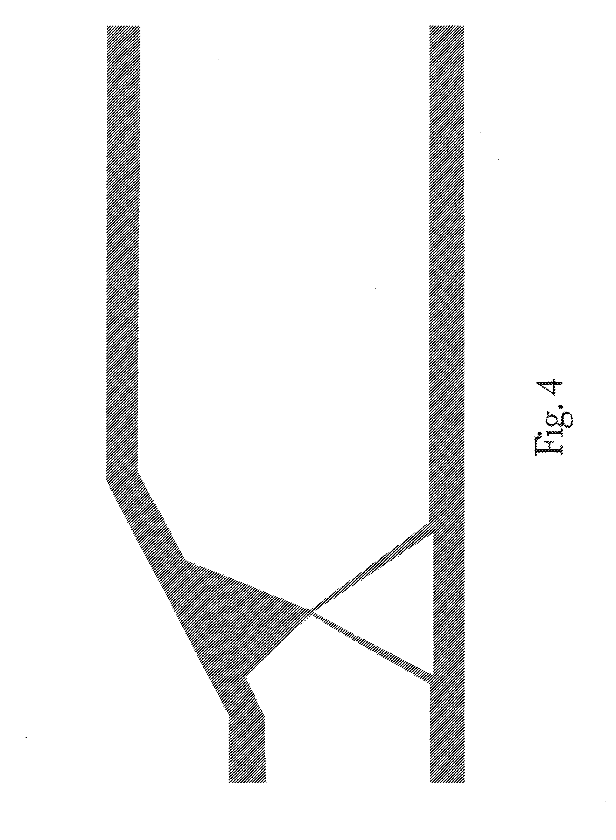

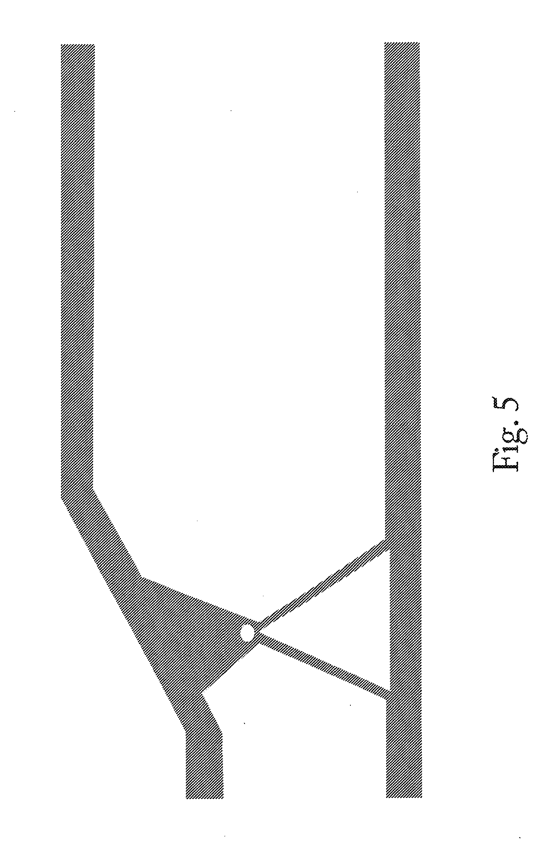

In den Figuren 4 und 5 ist eine alternative Ausführungsform des Werkzeugs mit zwei steifen Bauelementen, die mittels zweier nachgiebiger Verbindungselemente stoffschlüssig miteinander sind, wobei der Schnittpunkt der beiden Verbindungselemente auf einem vorgeschobenen Bereich des einen Bauelementes liegt. Diese Ausführungsform kann man so auffassen, dass der Schnittpunkt 18 der Verbindungselemente 14, 16; 22, 24 zwischen den beiden steifen Bauelementen 10, 12 liegt und die Verbindung zwischen dem Schnittpunkt 18 der Verbindungselemente 14, 16; 22, 24 und einem Bauelement 10 im Wesentlichen steif ausgestaltet ist oder aber so, dass die Verbindung eben einem der steifen Bauelemente zugerechnet wird. Insofern weist diese Ausführung lediglich eine konstruktive Variation auf, ohne die funktionalen Gegebenheiten der Erfindung zu verlassen.

Wenn - gemäss der Erfindung - bei dem Werkzeug der Hebel als Handgriff für die minimal invasive, insbesondere für die laparoskopische Chirurgie ausgebildet ist, dann kann die Bewegung des Hebels mittels einer Zug-/Druckstange - ganz allgemein durch ein geeignetes Übertragungselement - an das so genannte "distale" Ende des Instruments übertragen werden, das sich während des chirurgischen Eingriffs im menschlichen Körper befindet. Das "distale" Ende kann in diesem Ausführungsbeispiel je nach Zweck verschiedene Formen annehmen. Typische distale Enden sind Nadelhalter, Scheren oder Zangen zur Entnahme von Gewebeproben.

### Bezugszeichenliste

- 10: erstes im Wesentlichen steifes Bauelement
- 12: zweites im Wesentlichen steifes Bauelement
- 14: nachgiebiges Verbindungselement
- 16: nachgiebiges Verbindungselement
- 18: Schnittpunkt, Eckpunkt eines Dreieckes
- 22: nachgiebiges Verbindungselement
- 24: nachgiebiges Verbindungselement
- 26: nachgiebiges Verbindungselement
- 28: Schnittpunkt, Eckpunkt eines Dreieckes
- 30: Bohrungen

## Patentansprüche

1. Handgriff für die minimal invasive Chirurgie, insbesondere laparoskopische Chirurgie,zum Übertragen von Kräften und/oder Bewegungen, mit zumindest zwei, im Wesentlichen steifen Bauelementen (10, 12), wobei die beiden Bauelemente (10, 12) mittels zumindest zweier nachgiebiger Verbindungselemente (14, 16; 22, 24) stoffschlüssig miteinander so verbunden sind, dass sie gegeneinander durch Krafteinwirkung bewegbar sind,
**dadurch gekennzeichnet, dass**
sich die Mittellinien der Verbindungselemente (14, 16; 22, 24) in einem Punkt (18) schneiden,
wobei der Schnittpunkt (18) auf der Oberfläche eines ersten (10) der im Wesentlichen steifen Bauelemente, oder zumindest in einem kleinen Abstand dazu, liegt und die Verbindungselemente (14,16; 22, 24) jeweils mit dem anderen Bauelement (12) verbunden sind, wobei der genannte Schnittpunkt (18) der Mittellinien der Verbindungselemente (14, 16; 22, 24) in einem Abstand von maximal dem Zehnfachen der dünnsten Breite der Verbindungselemente (14, 16; 22, 24), vorzugsweise dem Fünffachen der dünnsten Breite der Verbindungselemente (14, 16; 22, 24), höchst vorzugsweise dem Doppelten der dünnsten Breite der Verbindungselemente (14, 16; 22, 24) zum ersten Bauelement (10) liegt,
und wobei eine Zug-/Druckstange zum Übertragen der Bewegung des Handgriffs an ein distales Ende eines Instruments vorgesehen ist.

2. Handgriff nach Anspruch 1, **dadurch gekennzeichnet, dass** der Schnittpunkt (18) im Bereich des ersten Bauelementes (10) liegt.

3. Handgriff nach Anspruch 1, **dadurch gekennzeichnet, dass** der Schnittpunkt (18) der Verbindungselemente (14, 16; 22, 24) zwischen den zwei steifen Bauelementen (10, 12) liegt und die Verbindung zwischen dem Schnittpunkt (18) der Verbindungselemente (14, 16; 22, 24) und einem Bauelement (10) im Wesentlichen steif ausgestaltet ist.

4. Handgriff nach einem der Ansprüche 1 bis 3, **gekennzeichnet durch** weitere nachgiebige Verbindungselemente (26), wobei jeweils zwei der Verbindungselemente (22, 24, 26) mit einem Teilstück eines der Bauelemente derart ein Dreieck bilden, dass jeweils eine der Ecken (18, 28) des Dreieckes auf der Oberfläche des ersten Bauelementes (10) oder zumindest in einem kleinen Abstand dazu liegen, wobei jeweils eine der Ecken (18, 28) der Dreiecke in einem Abstand von maximale dem Zehnfachen der dünnsten Breite der Verbindungselemente (22, 24, 26), vorzugsweise dem Fünffachen der dünnsten Breite der Verbindungselemente (22, 24, 26), höchst vorzugsweise dem Doppelten der dünnsten Breite der Verbindungselemente (22, 24, 26) zum ersten Bauelement (10) liegt.

## Claims

1. A handle for minimally invasive surgery, in particular laparoscopic surgery, for transferring forces and/or movements, with at least two mainly rigid structural elements (10, 12), wherein the two structural elements (10, 12) are interconnected in a surface-bonded manner by means of at least two flexible connecting elements (14, 16; 22, 24) such that they can be moved against each other through the application of force, **characterized in that**
the center lines of the connecting elements (14, 16; 22, 24) intersect at a point (18), wherein the point of intersection (18) lies on the surface of a first (10) of the mainly rigid structural elements, or at least at a short distance from it, and the connecting elements (14, 16; 22, 24) are respectively connected with the other structural element (12), wherein the named point of intersection (18) of the center lines of the connecting elements (14, 16; 22, 24) lies at a distance of a maximum of ten times the thinnest width of the connecting elements (14, 16; 22, 24), preferably five times the thinnest width of the connecting elements (14, 16; 22, 24), most preferably twice the thinnest width of the connecting elements (14, 16; 22, 24) from the first structural element (10),
and wherein a pull/push rod is provided for transferring the movement of the handle to a distal end of an instrument.

2. The handle according to claim 1, **characterized in that** the point of intersection (18) lies in the area of the first structural element (10).

3. The handle according to claim 1, **characterized in that** the point of intersection (18) of the connecting elements (14, 16; 22, 24) lies between the two rigid structural elements (10, 12) and the connection between the point of intersection (18) of the connecting elements (14, 16; 22, 24) and a structural element (10) is developed mainly rigidly.

4. The handle according to one of claims 1 to 3, **characterized by** additional flexible connecting elements (26), wherein respectively two of the connecting elements (22, 24, 26) form a triangle with a section of one of the structural elements such that respectively one of the corners (18, 28) of the triangle lies on the surface of the first structural element (10) or at least at a small distance from it, wherein respectively one of the corners (18, 28) of the triangles lies at a distance of a maximum of ten times the thinnest width of the connecting elements (22, 24, 26), preferably five times the thinnest width of the connecting elements (22, 24, 26), most preferably twice the thinnest width of the connecting elements (22, 24, 26) from the first structural element (10).

## Revendications

1. Poignée pour la chirurgie mini-invasive, en particulier la chirurgie laparoscopique, pour la transmission de forces et/ou de mouvements, avec au moins deux éléments de construction (10, 12) essentiellement rigides, les deux éléments de construction (10, 12) étant reliés entre eux par adhérence de matière à l'aide d'au moins deux éléments de liaison flexibles (14, 16 ; 22, 24), de manière à pouvoir se déplacer l'un par rapport à l'autre sous l'action d'une force,
**caractérisée en ce que**
les lignes médianes des éléments de liaison (14, 16 ; 22, 24) se croisent en un point (18), le point d'intersection (18) étant situé sur la surface d'un premier élément de construction (10) essentiellement rigide, ou du moins à une faible distance de celui-ci, et les éléments de liaison (14, 16 ; 22, 24) sont respectivement reliés à l'autre élément de construction (12), ledit point d'intersection (18) entre les lignes médianes des éléments de liaison (14, 16 ; 22, 24) étant situé à une distance maximale correspondant à dix fois la largeur la plus fine des éléments de liaison (14, 16 ; 22, 24), de préférence cinq fois la largeur la plus fine des éléments de liaison (14, 16 ; 22, 24), de la façon la plus préférentielle deux fois la largeur la plus fine des éléments de liaison (14, 16 ; 22, 24), par rapport au premier élément de construction (10), et dans laquelle il est prévu une barre de traction/pression pour la transmission du mouvement de la poignée à une extrémité distale d'un instrument.

2. Poignée selon la revendication 1, **caractérisée en ce que** le point d'intersection (18) se trouve dans la région du premier élément de construction (10).

3. Poignée selon la revendication 1, **caractérisée en ce que** le point d'intersection (18) des éléments de liaison (14, 16 ; 22, 24) se situe entre les deux éléments de construction rigides (10, 12), et la liaison entre le point d'intersection (18) des éléments de liaison (14, 16 ; 22, 24) et un élément de construction est conçue de façon essentiellement rigide.

4. Poignée selon l'une des revendications 1 à 3, **caractérisée par** d'autres éléments de liaison flexibles (26), dans laquelle deux des éléments de liaison (22, 24, 26) forment respectivement un triangle avec une partie de l'un des éléments de construction, de manière à ce que l'un des coins (18, 28) du triangle se trouve respectivement sur la surface du premier élément de construction (10) ou du moins à une faible distance de celui-ci, l'un des coins (18, 28) des triangles étant situé à une distance maximale correspondant à dix fois la largeur la plus fine des éléments de liaison (22, 24, 26), de préférence cinq fois la largeur la plus fine des éléments de liaison (22, 24, 26), de la façon la plus préférentielle deux fois la largeur la plus fine des éléments de liaison (22, 24, 26), par rapport au premier élément de construction (10).
